(19) Europäisches Patentamt
European Patent Office
Office européen des brevets

(11) Veröffentlichungsnummer: **0 495 137 A1**

# (12) EUROPÄISCHE PATENTANMELDUNG

(21) Anmeldenummer: **91100415.8**

(22) Anmeldetag: **15.01.91**

(51) Int. Cl.5: **A61B 6/03**, A61B 6/04

(43) Veröffentlichungstag der Anmeldung:
**22.07.92 Patentblatt 92/30**

(84) Benannte Vertragsstaaten:
**DE FR GB**

(71) Anmelder: **SIEMENS AKTIENGESELLSCHAFT**
**Wittelsbacherplatz 2**
**W-8000 München 2(DE)**

(72) Erfinder: **Kalender, Willi, Dipl.-Phys. Dr.**
**Auf der Höh 5**
**W-8521 Kleinseebach(DE)**

(54) **Computertomograph.**

(57) Es soll ein Computertomograph Geschaffen werden, der eine Untersuchung eines stehenden Patienten bei praktikablen, d.h. sehr kurzen Gesamtuntersuchungszeiten ermöglicht.

Die Patientenlagerung (21) ist zur Aufnahme das Patienten in aufrechter Position ausgebildet und es ist eine Relativvarstellung zwischen Patientenlagerung (21) und Meßsystem (19) in vertikaler Richtung möglich. Die Bewegung des Fokus des Meßsystems (19) auf einer Kreisbahn um das Meßfeld kann durch elektronische Ablenkung erfolgen.

EP 0 495 137 A1

Bei allen heute verfügbaren Computertomographen wird der Patient liegend, d.h. meist in Rückenlage, manchmal auch in Bauchlage, untersucht. Dies stellt in vielen Fällen die einzige Möglichkeit dar, da der Patient, bedingt durch Krankheit oder Trauma, häufig nicht aufrecht stehen kann. Häufig erfolgt die liegende Untersuchung aber auch deshalb, weil die Untersuchungen im Bereich von 15 bis 30 Minuten oder auch länger nur in entspannter, liegender Position durchgeführt werden können, um Bewegungen zu reduzieren oder zu vermeiden. Bei einigen Untersuchungen wäre es aber vorteilhaft, den Patienten in seiner normalen, aufrechten Position zu untersuchen. Dies wäre insbesondere für Lungenuntersuchungen besonders interessant. Die übliche Thoraxübersichtsaufnahme, die eine der häufigsten Röntgenuntersuchungen darstellt, wird vorzugsweise im Stehen angefertigt. Es ist wünschenswert, daß diese Untersuchung durch die sehr viel empfindlichere computertomographische Untersuchung ersetzt oder ergänzt wird.

Der Erfindung liegt die Aufgabe zugrunde, einen Computertomographen zu schaffen, der Untersuchungen am stehenden Patienten ermöglicht.

Diese Aufgabe ist erfindungsgemäß dadurch gelöst, daß die Patientenlagerung zur Aufnahme des Patienten in aufrechter Position ausgebildet ist und eine Relativverstellung zwischen Patientenlagerung und Meßsystem zur Abtastung des Patienten in vertikaler Richtung vorgesehen ist. Dabei ist es möglich, ein elektronisch arbeitendes Meßsystem zu verwenden, bei dem eine Gesamtaufnahmezeit im Bereich von einer Minute und weniger resultiert, so daß die Untersuchung ähnlich wie eine Standard-Röntgenfilmaufnahme durchgeführt werden kann. Aufgrund der Möglichkeit der Relativverstellung ist eine spiralförmige Abtastung eines Volumens des Patienten möglich, wobei Pausen zwischen den einzelnen Abtastvorgängen entfallen. Für den erfindungsgemäßen Computertomographen eignet sich insbesondere ein Meßsystem, bei dem die Bewegung des Fokus auf einer Kreisbahn um das Meßfeld durch elektronische Ablenkung erfolgt. Dadurch werden besonders kurze Meßzeiten erzielt.

Die Erfindung ist nachfolgend anhand eines in der Zeichnung dargestellten Ausführungsbeispieles näher erläutert. Es zeigen:

Fig. 1    das Meßsystem eines Computertomographen nach der Erfindung, und

Fig. 2    einen Computertomographen nach der Erfindung mit dem Meßsystem gemäß Fig. 1.

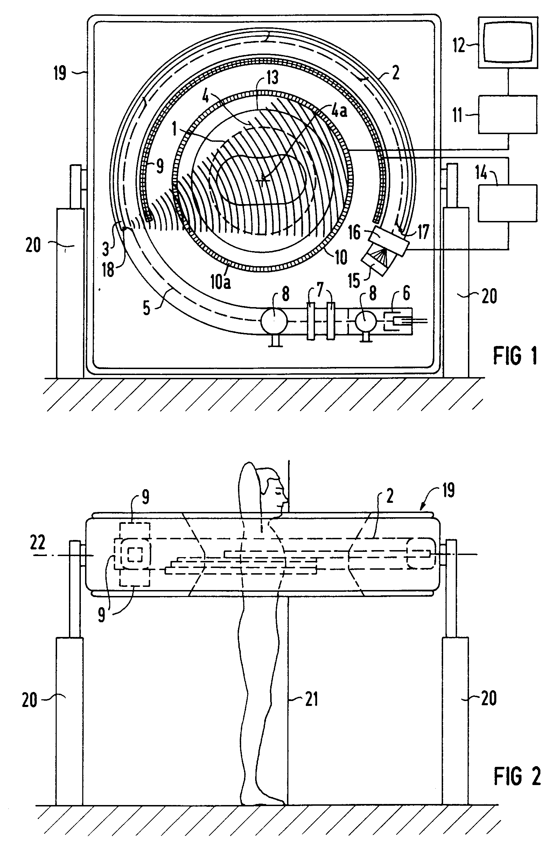

In der Fig. 1 ist das Meßsystem eines Röntgencomputertomographen mit einer ein Meßfeld 1 umgebenden, ringförmigen Röntgenstrahlenquelle 2 dargestellt, in der eine Ringanode 3 angeordnet ist. Die Ringanode 3 wird zur Erzeugung eines sich drehenden, fächerförmigen Röntgenstrahlenbündels 4 von einem Elektronenstrahl 5 abgetastet, der von einer Elektronenkanone 6 erzeugt wird. Der Elektronenkanone 6 sind Fokussierungsspulen 7 nachgeschaltet. Das Vakuum in der Röntgenstrahlenquelle 2 wird durch Vakuumpumpen 8 aufrecht erhalten. Der Elektronenstrahl 5 wird zur Erzeugung des Röntgenstrahlenbündels 4 mit Hilfe einer magnetischen Ablenkspule 9 auf die Ringanode 3 abgelenkt. Die aus dem Untersuchungsobjekt im Meßfeld 1 austretende Röntgenstrahlung wird von einem ringförmigen Strahlendetektor 10, der aus einer Reihe von Detektorelementen 10a besteht, erfaßt. Die Ausgangssignale der Detektorelemente 10a usw. werden einem Rechner 11 zugeführt, der daraus ein Bild der untersuchten Schicht des Untersuchungsobjektes berechnet und auf einem Monitor 12 wiedergibt. Das Meßfeld 1 ist ein Feld in einer Öffnung 13, in die das Untersuchungsobjekt eingeschoben wird. Das Röntgenstrahlenbündel 4 rotiert durch Ablenkung des Elektronenstrahles 5 auf die Ringanode 3 zur Durchstrahlung des Untersuchungsobjektes unter verschiedenen Richtungen um die Achse 4a.

Eine Steuervorrichtung 14 steuert die Ablenkspule 9 in der Weise, daß der Elektronenstrahl 5 vor Beginn eines Abtastvorganges die Röntgenstrahlenquelle 2 konzentrisch zur Ringanode 3 durchsetzt, bis er am geschlossenen Ende auf einem Strahlenfänger 15, z.B. aus Blei, auftrifft. Vorher wird er durch eine Defokussiereinrichtung 16 defokussiert. Anschließend wird er durch die Ablenkspule 9 auf die Ringanode 3 abgelenkt und tastet diese von ihrem Ende 17 zu ihrem Ende 18 ab. Fünf Fokuspositionen sind in der Zeichnung dargestellt. Tatsächlich sind es wesentlich mehr diskrete Fokuspositionen, z.B. 1 000. Vorzugsweise soll der Fokus aber durch ein Wanderfeld kontinuierlich verschoben werden, so daß die Abtastung über die Detektorabfrage festgelegt wird. Das Röntgenstrahlenbündel 4 dreht sich also entgegen der Richtung des Elektronenstrahles 5 und befindet sich in der Zeichnung in seiner Endstellung. Hier ist ein Abtastvorgang beendet.

Danach erfolgt ein erneuter Aufbau des ringförmig geführten Elektronenstrahles 5. Mit dessen Ablenkung auf das Ende 17 der Ringanode 3 beginnt ein neuer Abtastvorgang.

Es ist auch möglich, die Ringanode 3 durch den Elektronenstrahl 5 im Uhrzeigersinn, also von ihrem Ende 18 zu ihrem Ende 17, abzutasten.

Der Strahlendetektor 4 ist hinsichtlich der Ringanode 3 so angeordnet, daß das Röntgenstrahlenbündel an ihm vorbei passieren kann, bevor es in das Meßfeld 1 eintritt und erst nach seinem Austritt aus dem Meßfeld 1 auf dem Strahlendetektor 10 auftrifft.

Aus der Fig. 2 geht hervor, daß das Meßsystem 19 das Computertomographen mit dar Röntgenstrahlenquelle 2 in vertikaler Richtung verstellbar an Stützen 20 gelagert ist. Ferner zeigt die Fig. 2, daß eine Lagerungsplatte 21 bzw. Fläche zum Anlehnen oder Fixieren für den Patienten zu dessen Lagerung in aufrechter Position vorgesehen ist. Computertomogramme können also durch entsprechende Einstellung des Meßsystems 19 (Schwenkung um die horizontale Achse 22) bei stehendem Patienten angefertigt werden. Die jeweils untersuchte Schicht wird dabei durch Einstellung das Maßsystems 19 in vertikaler Richtung gewählt. Wird das Meßsystem 19 langsam kontinuierlich in vertikaler Richtung verstellt und dabei der Patient abgetastet, so erhält man eine spiralförmige Abtastung eines vorgegebenen Patientenvolumens.

Das Meßsystem 19, bei dem die Bewegung des Fokus der Röntgenstrahlenquelle 2 auf einer Kreisbahn, nämlich auf der Anode 3, um das Meßfeld 1 durch elektronische Ablenkung erfolgt, ist um die horizontale Achse 22 schwenkbar, so daß auch Schrägabtastungen unter beliebigen Winkeln möglich sind.

**Patentansprüche**

1. Computertomograph mit einem Maßsystem (19) zur Abtastung das Patienten und einer Patientenlagerung (21), bei dem die Patientenlagerung (21) zur Aufnahme des Patienten in aufrechter Position ausgebildet ist und eine Relativverstellung zwischen Patientenlagerung (21) und Meßsystem (19) in vertikaler Richtung vorgesehen ist.

2. Computertomograph nach Anspruch 1, bei dem die Bewegung des Fokus auf einer Kreisbahn um das Meßfeld (1) durch elektronische Ablenkung erfolgt.

3. Computertomograph nach Anspruch 1 oder 2, bei dem das Meßsystem (19) um eine horizontale Achse (22) schwenkbar ist.

FIG 1

FIG 2

Europäisches
Patentamt

**EUROPÄISCHER RECHERCHENBERICHT**

Nummer der Anmeldung

EP 91 10 0415

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 298 800 (A. GOLDMAN) * Spalte 6, Zeilen 30-38; Spalte 9, Zeile 42 - Spalte 10, Zeile 39; Abbildung 2 * --- | 1 | A 61 B 6/03 A 61 B 6/04 |
| A | GB-A-2 044 985 (EMI LTD) * Zusammenfassung; Seite 1, Zeilen 28-65; Abbildungen 1a,1b,2 * --- | 2 | |
| A | GB-A-2 228 168 (HAMAMATSU PHOTONICS K.K.) * Seite 2, Zeile 22 - Seite 3, Zeile 9; Seite 4, Zeile 20 - Seite 5, Zeile 20; Abbildung 1 * ----- | 1,3 | |
| | | | **RECHERCHIERTE SACHGEBIETE (Int. Cl.5)** A 61 B |

Der vorliegende Recherchenbericht wurde für alle Patentansprüche erstellt

| Recherchenort | Abschlußdatum der Recherche | Prüfer |
|---|---|---|
| DEN HAAG | 27-08-1991 | FONTENAY P.H.E.V. |